(19) Europäisches Patentamt
European Patent Office
Office européen des brevets

(11) Publication number: **0 330 025 B1**

# (12) EUROPEAN PATENT SPECIFICATION

(45) Date of publication of patent specification: **12.10.94** (51) Int. Cl.5: **A61K 31/557**

(21) Application number: **89102374.9**

(22) Date of filing: **11.02.89**

## (54) Antidiabetic composition comprising isocarbacyclin derivative.

(30) Priority: **23.02.88 JP 38742/88**
**16.09.88 JP 230093/88**

(43) Date of publication of application:
**30.08.89 Bulletin 89/35**

(45) Publication of the grant of the patent:
**12.10.94 Bulletin 94/41**

(84) Designated Contracting States:
**AT BE CH DE ES FR GB IT LI NL SE**

(56) References cited:

**EP-A- 0 229 844**

**PATENT ABSTRACTS OF JAPAN, vol. 11, no. 24 (C-399)[2471], 23rd January 1987; & JP-A-61 197 518 (TEIJIN LTD) 01-09-1986**

**THE LANCET, vol. 2, no. 8446, 13th July 1985, pages 95-96; R. CHIESA et al.: "Use of stable prostacyclin analogue ZK 36 374 to treat severe lower limb ischaemia"**

**DIALOG INFORMATION SERVICES, file 155, MEDLINE, accession no. 06580775; J.A. COLWELL et al.: "Do platelets have anything to do with diabetic microvascular disease?"**

(73) Proprietor: **TEIJIN LIMITED**
**6-7, Minamihonmachi 1-chome**
**Chuo-ku**
**Osaka-shi Osaka 541 (JP)**

Proprietor: **FUJISAWA PHARMACEUTICAL CO., LTD.**
**4-7, Doshomachi 3-chome**
**Chuo-ku**
**Osaka-shi Osaka 541 (JP)**

(72) Inventor: **Tanaka, Toshio**
**17-20, Tamadaira 6-chome**
**Hino-shi Tokyo 191 (JP)**
Inventor: **Horiai, Haruo**
**1-1-306, Mukonoso 3-chome**
**Amagasaki-shi Hyogo 661 (JP)**

(74) Representative: **Türk, Gille, Hrabal, Leifert**
**Brucknerstrasse 20**
**D-40593 Düsseldorf (DE)**

EP 0 330 025 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

1. Field of the Invention

The present invention relates to the use of an antidiabetic composition comprising an isocarbacyclin derivative as the active ingredient, for the manufacture of a medicament for therapeutic treatment of diabetic neuropathy.

2. Description of the Prior Art

Prostaglandins have various physiological activities such as potent platelet aggregation inhibitory activity, vasodilating activity, hypotensive activity, gastric juice secretion inhibitory activity, smooth muscle contracting activity and diuretic activity and are substances which are useful for curing or preventing peripheral circulation disorders, myocardial infarction, angina pectoris, arteriosclerosis, hypertension, gastric ulcer, duodenal ulcer, etc.

Of these prostaglandins, prostaglandins $E_1$ have recently attracted attention in respect of effects to improve peripheral circulation through their platelet aggregation inhibitory activity and vasodilating activity. Such compounds are clinically found useful for curing various diseases including Buerger's disease, arteriosclerosis obliterans and like arteriostenosis, ischemic ulcer and diabetic gangrene. Of these diseases, diabetic gangrene, which is one of the complications of diabetes, is thought to be induced by the participation of factors associated with peripheral arteriosclerosis, diabetic microangiopathy, diabetic neuropathy, opportunistic infections and the like. Reports are made as to the efficacy of prostaglandins $E_1$ on diabetic gangrene (see Haruhiko Ninomiya et al., "Modern Medical Care," 15, 710-712, 1983; Ryuji Sano et al., "Modern Medical Care," 13, 142-148, 1981; Yasuhiro Oribe et al., "Diabetes," 24(8), 853-860, 1981; Haruhiko Nishima et al., "Prostaglandins-Advances in clinical applications II," Gendai Iryosha, 228-231, 1985; Hiroyuki Hososhima et al., the same publication, 232-235, 1985; Nobuyuki Asakawa et al., the same publication, 236-241, 1985; Haruhito Nomoto et al., the same publication, 242-244, 1985; Hiroshi Hayashi et al., "Prostaglandins-Advances in Clinical applications," Gendai Iryosha, 165-173, 1983; and literature citing these reports). Effective cases of prostaglandins $E_1$ for diabetic neuropathy have also been recently reported (see Tsuguo Ebihara et al., "Prostaglandins-Advances in Clinical Applications II," Gendai Iryosha, 245-248, 1985; Kenshin Kishida et al., the same publication, 249-256, 1985; Fumio Umeda et al., the same publication, 257-262, 1985; Tsutomu Nakamura et al., the same publication, 263-268, 1985; Atsuhiko Tada et al., the same publication, 269-273, 1985; Yasuhiro Oribe et al., "Prostaglandins-Advances in Clinical Applications III," Gendai Iryosha, 145-148, 1985; Hisaji Kamoi et al., the same publication, 149-154, 1985; Hiroyasu Dohgen et al., the same publication, 155-160, 1985; Kazuaki Orita et al., the same publication, 161-166, 1985; Kiyoshi Hashizume et al., the same publication, 167-169, 1985; and literature citing these reports).

In these reports, prostaglandins $E_1$ are used all as intravenous drips, and nothing has been reported as to a successful therapy with oral administration. Also, it has not been reported that prostaglandin $I_2$ and derivative thereof are effective in the treatment of diabetes.

The inventors of the present invention have paid attention to the fact that when the human being is attacked with diabetes, the producibility of prostaglandin $I_2$ in vascular endothelial cells is lowered, and have conducted intensive studies on prostaglandin $I_2$ analogues which are orally effective on diabetes, especially diabetic neuropathy. Consequently, we found that isocarbacyclin derivatives, i.e., 9(0)-methano-$\Delta^{6(9\alpha)}$-prostaglandin $I_1$ derivatives represented by the below-mentioned formula [I] have the desired activity.

SUMMARY OF THE INVENTION

The present invention relates to an antidiabetic neuropathic composition comprising as the active ingredient an isocarbacyclin derivative of the formula [I]:

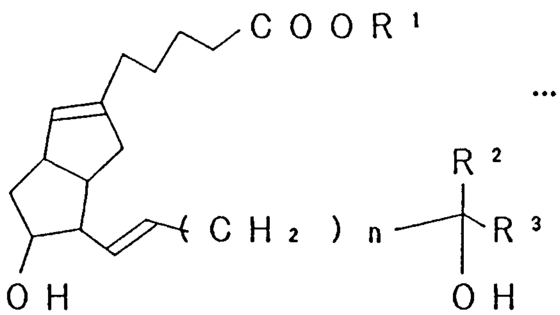

... [ I ]

wherein $R^1$ is hydrogen atom, an alkyl group or one equivalent of cation; $R^2$ is hydrogen atom or methyl group; $R^3$ is a straight-chain or branched-chain alkyl, alkenyl or alkynyl group, a cycloalkyl, phenyl or phenoxy group which may be substituted, or a straight-chain or branched-chain alkyl group which is substituted by the above mentioned cycloalkyl, phenyl or phenoxy group which may be substituted; n is 0 or 1, provided that when $R^3$ is n-pentyl, $R^2$ is methyl or n is 1.

DESCRIPTION OF THE PREFERRED EMBODIMENTS

In the formula [I], $R^1$ is hydrogen atom, an alkyl group or one equivalent of cation. The alkyl group means a straight-chain or branched-chain alkyl group, preferably having 1 to 10 carbon atoms, such as methyl, ethyl, n-propyl, isopropyl, n-butyl, sec-butyl, tert-butyl, n-pentyl, n-hexyl, n-heptyl, n-octyl, n-nonyl, n-decyl, etc. Examples of useful cations (one equivalent) are alkali metal cations such as $Na^+$ and $K^+$, bivalent or trivalent metal cations such as 1/2 $Ca^{2+}$, 1/2 $Mg^{2+}$ and 1/3 $Al^{3+}$, ammonium cations such as ammonium ion and tetramethylammonium ion. Especially preferably as $R^1$ is hydrogen atom or methyl.

$R^2$ in the formula [I], which represents hydrogen atom or methyl, is preferably hydrogen when n is 0, or methyl when n is 1.

$R^3$ in the formula [I] is a straight-chain or branched-chain alkyl group, preferably having 3 to 10 carbon atoms; a straight-chain or branched-chain alkenyl group, preferably having 3 to 10 carbon atoms; a straight-chain or branched-chain alkynyl group, preferably having 3 to 10 carbon atoms; a cycloalkyl group, preferably having 3 to 10 carbon atoms, which may be substituted; a phenoxy group which may be substituted; a phenyl group which may be substituted; or a straight-chain or branched-chain alkyl group, preferably having 1 to 5 carbon atoms, which is substituted by the above-mentioned phenyl, phenoxy or cycloalkyl group which may be substituted.

Examples of the straight-chain or branched-chain alkyl groups include n-propyl, n-butyl, n-pentyl, n-hexyl, n-heptyl, n-octyl, n-decyl, 1-methylpentyl, 1-methylhexyl, 1,1-dimethylpentyl, 2-methylpentyl, 2-methylhexyl, 5-methylhexyl, 2,5-dimethylhexyl, etc. preferably n-butyl, n-pentyl, n-hexyl, (R)-, (S) or (RS)-1-methylpentyl and (R)-, (S)-or (RS)-2-methylhexyl.

Examples of the straight-chain or branched-chain alkenyl groups include 2-butenyl, 2-pentenyl, 3-pentenyl, 2-hexenyl, 4-hexenyl, 2-methyl-4-hexenyl, 2,6-dimethyl-5-heptenyl, etc.

Examples of the straight-chain or branched-chain alkynyl groups include 2-butynyl, 2-pentynyl, 3-pentynyl, 2-hexynyl, 4-hexynyl, 2-octynyl, 5-decynyl, 1-methyl-3-pentynyl, 1-methyl-3-hexynyl, 2-methyl-4-hexynyl, etc.

Examples of the cycloalkyl group which may be substituted include cyclopropyl, cyclobutyl, cyclopentyl, cyclohexyl, cycloheptyl, cyclooctyl, $C_{1-5}$ alkylcyclopentyl $C_{1-4}$ alkylcyclohexyl, dimethylcyclopentyl, dimethylcyclohexyl, chlorocyclopentyl, bromocyclohexyl, iodocyclopentyl, fluorocyclohexyl, etc. The preferred one is cyclopentyl or cyclohexyl.

Examples of substituents of the phenyl or phenoxy group which may be substituted include a halogen, hydroxy, a $C_{2-7}$ acyloxy, a $C_{1-4}$ alkyl optionally substituted by a halogen, a $C_{1-4}$ alkoxy optionally substituted by a halogen; cyano, carboxy, a $C_{1-6}$ alkoxycarbonyl, etc. The halogen may be fluorine, chlorine, bromine or the like, preferably fluorine or chlorine. Preferably, the $C_{2-7}$ acyloxy may be acetoxy, propionyloxy, n-butyryloxy, n-valeryloxy, iso-valeryloxy, caproyloxy, enanthyloxy, benzoyloxy or the like. Preferably, the $C_{1-4}$ alkyl optionally substituted by a halogen may be methyl, ethyl, n-propyl, iso-propyl, n-butyl, chloromethyl, dichloromethyl, trifluoromethyl or the like. Preferably, the $C_{1-4}$ alkoxy optionally substituted by a halogen may be methoxy, ethoxy, n-propoxy, iso-propoxy, n-butoxy, chloromethoxy, dichloromethoxy, trifluoromethoxy or the like. The $C_{1-6}$ alkoxycarbonyl may be methoxycarbonyl, ethoxycarbonyl, butoxycarbonyl, hexyloxycarbonyl or the like. The phenyl or phenoxy group may be substituted by one to three, preferably one, of the above mentioned substituents.

The above mentioned phenyl or phenoxy groups which may be substituted are suitably applicable to those in the straight-chain or branched-chain alkyl group (preferably having 1 - 5 carbon atoms) which is substituted by a phenyl or phenoxy which may be substituted or a cycloalkyl (preferably having 3 - 10 carbon atoms) which may be substituted. The above mentioned cycloalkyl groups which may be substituted are also suitably applicable to that in the straight-chain or branched-chain alkyl group. Examples of substituents on the phenyl, phenoxy or cycloalkyl group are the same ones of the above mentioned substituted phenyl, phenoxy or cycloalkyl group. Examples of the straight-chain or branched chain $C_{1-5}$ alkyl groups include methyl, ethyl, propyl, isopropyl, n-butyl, isobutyl, sec-butyl, t-butyl, pentyl, etc., on which substituent(s) may be bonded at optional position.

Especially, $R^3$ is preferred to be n-butyl, n-pentyl, 1-methylpentyl, 2-methylhexyl, cyclopentyl, cyclohexyl, 2,6-dimethyl-5-heptenyl, 1-methyl-3-pentenyl, 1-methyl-3-hexynyl or the like. However, when $R^3$ is n-pentyl, $R^2$ should be methyl or n should be 1. That is in case where $R^2$ is hydrogen, $R^3$ is n-pentyl and n is 0, the side chain moiety constituted by such case is the quite same as that in natural prostaglandins which are bio-synthesized from arachidonic acid as substrate in the living body. Therefore, the compound having such side chain moiety is rapidly metabolized, in a similar way for natural prostaglandins, in the living body and hence is hard to exert a lasting activity when it is orally administered.

In the formula [I], n is 0 or 1. In case where n is 0, the compounds of the formula [I] contain one asymmetric carbon to which the substituents $R^2$ and $R^3$, and hydroxy group are bonded. Thus, this invention includes the compounds of the formula [I-1] having natural type configuration at the 15th position,

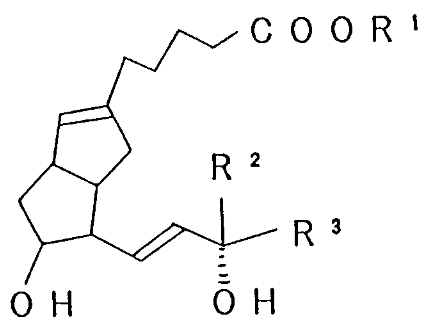

... [ I - 1 ]

wherein $R^1$, $R^2$ and $R^3$ are the same as defined above, the compounds of the formula [I-2] having non-natural type configuration at 15th position,

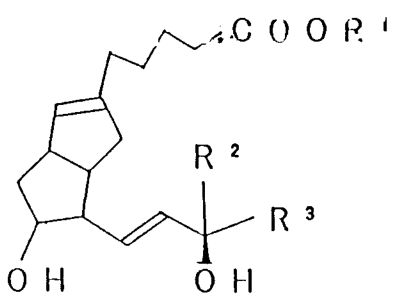

... [ I - 2 ]

wherein $R^1$, $R^2$ and $R^3$ are the same as defined above, and mixtures thereof in an optional ratio.

Especially, when $R^2$ is hydrogen, it is preferred to be the compounds of the above formula [I-1] having the 15th natural type configuration.

When n is 1, this invention includes the compounds of the 16th (R)-configuration and the 16th (S)-configuration and mixtures thereof in an optional ratio.

Also, other stereoisomers exist in the isocarbacyclin derivatives of the formula [I], because they contain four kinds of asymmetric carbon atoms on the bicyclo [3.3.0] octene ring as nucleus thereof (corresponding to carbon atoms at 8th, 9th, 11th and 12th positions according to the prostaglandine nomenclature).

Isocarbacyclin derivatives represented by the formula [I-3]

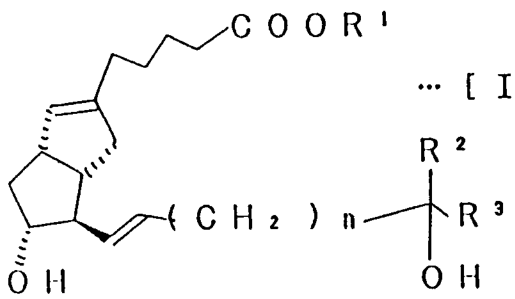

... [ I - 3 ]

, wherein the definitions of $R^1$, $R^2$, $R^3$ and n are the same as those defined above, are especially useful stereoisomers, because their configuration is the same as natural prostaglandins. Of course, this invention includes other stereoisomers than the above formula [I-3] and mixtures thereof in an optional ratio.

Preferred examples of the isocarbacyclin [9(0)-methano-$\Delta^{6(9\alpha)}$-prostaglandin $I_1$] derivatives to be used in the diabetic therapeutic composition are as follows.

(1) 20-methyl-9(0)-methano-$\Delta^{6(9\alpha)}$-prostaglandin $I_1$
(2) 16-methyl-9(0)-methano-$\Delta^{6(9\alpha)}$-prostaglandin $I_1$
(3) 16, 16-dimethyl-9(0)-methano-$\Delta^{6(9\alpha)}$-prostaglandin $I_1$
(4) 17-methyl-9(0)-methano-$\Delta^{6(9\alpha)}$-prostaglandin $I_1$
(5) 17, 20-dimethyl-9(0)-methano-$\Delta^{6(9\alpha)}$-prostaglandin $I_1$
(6) (17R)-isomer of (5)
(7) (17S)-isomer of (5)
(8) 15-methyl-9(0)-methano-$\Delta^{6(9\alpha)}$-prostaglandin $I_1$
(9) 17, 18-dehydro-9(0)-methano-$\Delta^{6(9\alpha)}$-prostaglandin $I_1$
(10) 20-isopropylidene-17-methyl-9(0)-methano-$\Delta^{6(9\alpha)}$-prostaglandin $I_1$
(11) 18,18,19,19-tetradehydro-16-methyl-9(0)-methano-$\Delta^{6(9\alpha)}$-prostaglandin $I_1$
(12) 18,18,19,19-tetradehydro-16,20-dimethyl-9(0)-methano-$\Delta^{6(9\alpha)}$-prostaglandin $I_1$
(13) (16S)-isomers of (2), (11) and (12)
(14) (16R)-isomers of (2), (11) and (12)
(15) 16,17,18,19,20-pentanor-15-cyclopentyl-9(0)-methano-$\Delta^{6(9\alpha)}$-prostaglandin $I_1$
(16) 16,17,18,19,20-pentanor-15-cyclohexyl-9(0)-methano-$\Delta^{6(9\alpha)}$-prostaglandin $I_1$
(17) 17,18,19,20-tetranor-16-(p-fluorophenoxy)-9(0)-methano-$\Delta^{6(9\alpha)}$-prostaglandin $I_1$
(18) 17,18,19,20-tetranor-16-cyclohexyl-9(0)-methano-$\Delta^{6(9\alpha)}$-prostaglandin $I_1$
(19) 15-deoxy-16-hydroxy-9(0)-methano-$\Delta^{6(9\alpha)}$-prostaglandin $I_1$
(20) 15-deoxy-16-hydroxy-16-methyl-9(0)-methano-$\Delta^{6(9\alpha)}$-prostaglandin $I_1$
(21) (16S)-isomers of (19) and (20)
(22) (16R)-isomers of (19) and (20)
(23) methyl esters of (1) - (22)
(24) ethyl esters of (1) - (22)
(25) butyl esters of (1) - (22)
(26) sodium salts of (1) - (22)
(27) potassium salts of (1) - (22)
(28) ammonium salts of (1) - (22)
(29) enantiomers of (1) - (28)
(30) stereoisomers at 8th, 9th, 11th, 12th or 15th position of (1) - (28)

The above compounds are non-limitative examples.

The isocarbacyclin derivatives of the formula [I] can be easily prepared by the methods known in the art, e.g., those disclosed in Japanese Unexamined Patent Publication Nos. 59(1984)-210044 and 61(1986)-197518. They can be also prepared by our own method disclosed in Japanese Unexamined Patent Publication Nos. 62(1987)-87539 and 62(1987)-87540 i.e., the method shown by Scheme I.

Scheme I

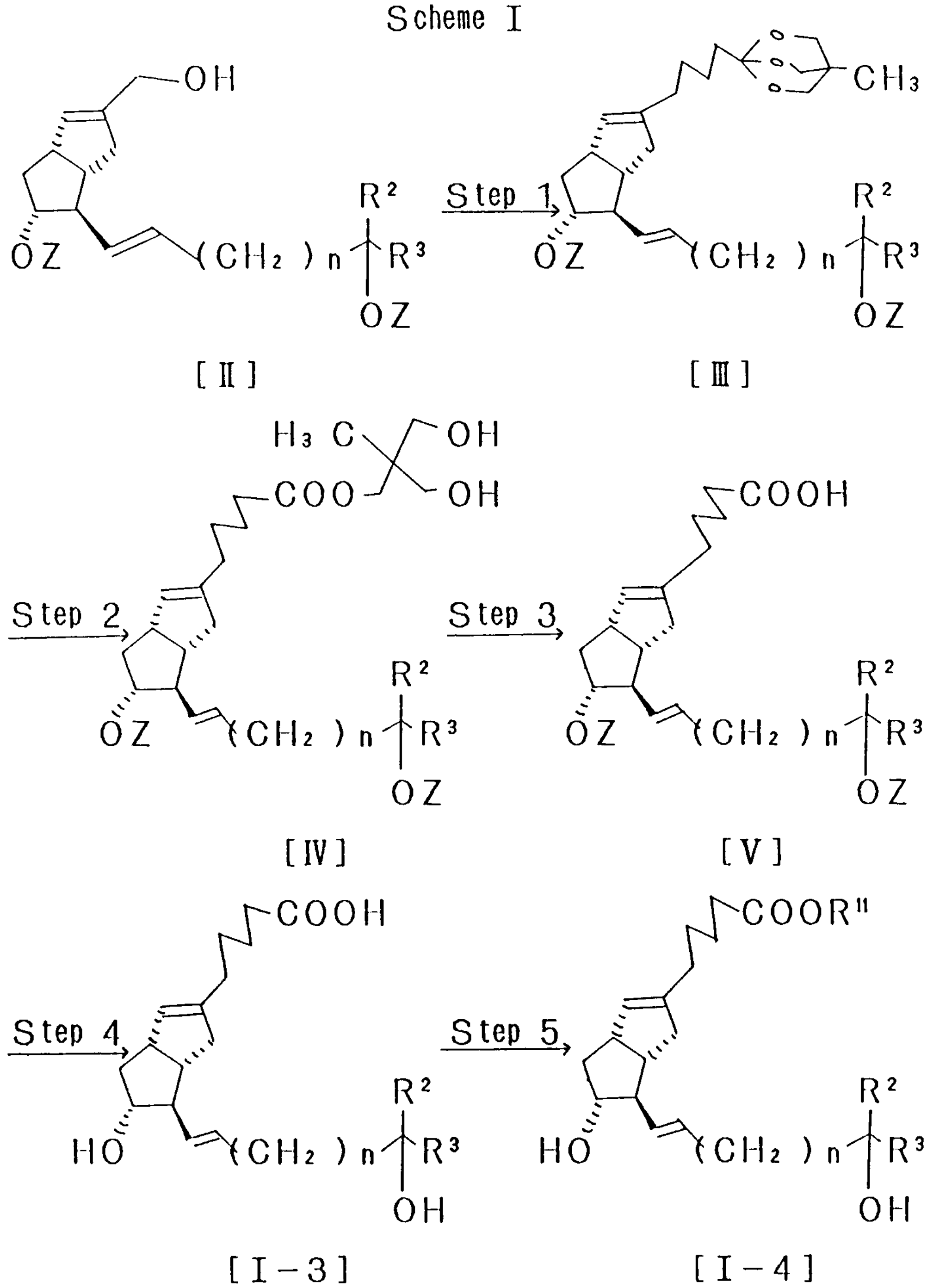


[II] [III] [IV] [V] [I-3] [I-4]

In the above Scheme, the definitions for $R^2$, $R^3$ and n in the compounds [II]-[V], [I-3] and [I-4] are the same as defined above, $R^{11}$ is an alkyl group preferably having 1-10 carbon atoms or one equivalent of cation and Z is t-butyldimethylsilyl group.

The step 1 in Scheme I concerns an alkylation, which can be conducted in accordance with Murahashi et al's method [J. Am. Chem. Soc., 99, p2361 (1977)]. An allyl alcohol [II] is reacted with n-butyllithium and then cuprous iodide, and further is reacted with 1-[3-lithiopropyl]-4-methyl-2,6,7-trioxabicyclo [2.2.2] octane of the formula [VI]

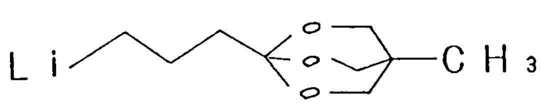
... [ VI ]

in the presence of N,N-methylphenylaminotriphenylphosphonium = iodide.

Besides, 1-[3-lithiopropyl]-4-methyl-2,6,7-trioxabicyclo [2.2.2] octane of the formula [VI] can be prepared by the reaction of 1-[3-bromopropyl]-4-methyl-2,6,7-trioxabicyclo [2.2.2] octane with t-butyl lithium [E. J. Corey et al: Tetrahedron Letters 24, p5571 (1983)].

The step 2 is a hydrolysis of the ortho ester and proceeds under the condition of the conventional hydrolysis on ortho esters using an acid catalyst. For example, the diol ester compound [IV] can be obtained from the ortho ester compound [III] by dissolving it in a mixture of methanol and water and adding to the solution a little amount of pyridinium salt of p-toluenesulfonic acid.

The step 3 is a hydrolysis of the ester which can be achieved by the conventional alkali hyrolysis method. For example, a solution of the diol ester compound [IV] in tetrahydrofuran is reacted with an aqueous solution of lithium hydroxide or sodium hydroxide, to afford the carboxylic acid compound [V].

The step 4 is a desilylation, which can be achieved by the conventional methods using a fluorine compound such as tetrabutylammonium fluoride or hydrogen fluoride, or an acid such as acetic acid or p-toluenesulfonic acid. For example, the disilyl compound [V] is reacted with tetrabutylammonium fluoride in tetrahydrofuran, to convert into the isocarbacyclin [I-3].

The step 5 is an esterification or salt-forming reaction which can be achieved in accordance with the conventional methods thereof. For example, the isocarbacyclin [I-3] is treated with diazomethane to yield the corresponding methyl ester, or with an alkyl iodide/diisopropylethylamine to yield the alkyl ester. A salt of the isocarbacyclin [I-3] can be also obtained by the conventional salt-forming reaction with e.g., sodium hydroxide or potassium hydroxide.

The enol compounds [II] as the starting materials of the method shown by Scheme I can be easily synthesized by the methods known in publications see, e.g., T. Mase et al: Tetrahedron Letters, 25, p5087 (1984).

Thus, the isocarbacyclin derivatives [I] per se are known compounds and are known to prevent or relieve thrombosis, angina pectoris, myocardinal infarction, arteriosclerosis, metastasis of malignant tumors, hypertension, hyperlipemia, organopathy and the like through their platelet aggregation inhibitory activity, hypotensive activity, vasodilating activity lipid lowering activity and cell protecting activity. Nevertheless, the present invention has revealed for the first time that these derivatives which belong to prostaglandin $I_2$ analogus exhibit diabetes curing activity.

For this purpose, the isocarbacyclin derivatives [I] can be given orally; or parenterally, e.g. intrarectally, subcutaneously, intramuscularly, intravenously or cutaneously. Preferably, these compounds are administered orally or intravenously.

For oral administration, the active ingredient can be made into solid preparations or liquid preparations. Examples of useful solid preparations are tablets, pellets, powder and granules. In formulating such solid preparations, the active ingredient is admixed with a pharmacologically acceptable carrier such as sodium bicarbonate, calcium carbonate, potato starch, sucrose, mannitol, carboxymethylcellulose or the like. While the preparation can be obtained by a conventional method, conjointly usable with such carriers are pharmaceutical additives such as lubricants including, for example, calcium stearate and magnesium stearate.

For example, an organic solvent or aqueous solution of an enteric substance, such as cellulose acetate phthalate, hydroxypropylmethylcellulose phthalate, polyvinyl alcohol phthalate, styrene-maleic anhydride copolymer or methacrylic acid-methyl methacrylate copolymer, can be sprayed onto the solid preparation to form an enteric coating thereon and obtain an enteric preparation. Powders, granules or like solid preparations can be enclosed with enteric capsules.

The liquid preparations for oral administration include, for example, emulsions, solutions, suspensions, syrups and elixirs. Such preparations contain a pharmacologically acceptable carrier which is generally used, e.g., water or liquid paraffin. Also usable as carriers are oily bases such as coconut oil, fractionated coconut oil, soybean oil and corn oil.

When required, the above mentioned preparations may contain auxiliary agent, perfume or flavoring agent; stabilizer or antiseptic which is usually used.

The liquid preparation may be given as encapsulated with an absorbable substance such as gelatin.

The solid preparation for intrarectal administration includes suppository containing the active ingredient and prepared by a known method.

The parenteral preparation is given in the form of aqueous or non-aqueous solution, suspension or emulsion as sterilized. The non-aqueous solution or suspension is prepared using a pharmacologically acceptable carrier such as propyl glycol, polyethylene glycol, olive oil or like vegetable oil, or ethyl oleate or like injectable organic ester. Auxiliary agents such as antiseptic, wetting agent, emulsifier, dispersant and stabilizer can be incorporated into such preparations. Such solution, suspension and emulsion can be

sterilized by a suitable treatment, for example, by filtration with a bacteria retaining filter, addition of an antiseptic, or irradiation with ultraviolet rays. It is also possible to prepare a sterilized solid preparation and dissolve the preparation in sterilized water or sterilized injectable solvent immediately before use.

For cutaneous administration, for example ointments are useful which are prepared by a usual method.

The isocarbacyclin derivatives [I] of the invention are usable also in the form of its inclusion compound with $\alpha$-, $\beta$- or $\gamma$-cyclodextrin, methylated cyclodextrin or the like.

The isocarbacyclin derivatives when to be used for curing diabetic neuropathy, can be administered usually at a daily dosage of about 1 $\mu$g to about 1 mg for adults although the dosage varies with the symptoms, age, sex and body weight of the patient, route of administration, etc. The compound can be given in a single dose, or in several divided doses, e.g. two to six divided doses, per day.

The present invention will be described below with reference to the following test examples and examples.

Test Example 1

Measurement for nerve conduction velocity in GK rats with spontaneous diabetes

A test substance, (17S)-17,20-dimethylisocarbacyclin [=(17S)-17,20-dimethyl-9(0)-methano-$\Delta^{6(9\alpha)}$-prostaglandin $I_1$] was used to observe any improvement effect on nervous disorder of GK rats with spontaneous diabetes.

At the test method was to use four groups, i.e., one group of Wistar rats as control, two groups of GK rats with spontaneous diabetes administered by the test substance and one group of the GK rats not administered by the test substance. Before testing, glucose tolerance and nerve conduction velocity were measured. Then the test substance was orally given for 4 weeks (one group at a dose of 30$\mu$g/Kg/day and another group at a dose of 300$\mu$g/Kg/day), and the animals were then checked for glucose tolerance and nerve conduction velocity. The results are shown in Table 1.

Table 1

Variations in nerve conduction velocity before administration of test substance and 4 weeks later

| Animals | | Nerve conduction velocity (m/sec) | |
|---|---|---|---|
| | Numbers | Before administration | 4 weeks later |
| Control (Wistar rats) | 10 | 36.5 ± 0.5 | 40.5 ± 0.8 |
| Control (GK rats) | 9 | 32.9 ± 0.8 | 36.4 ± 0.8 |
| GK rats 30μg/Kg/day | 9 | 33.7 ± 1.2 | 40.5 ± 0.8 |
| GK rats 300μg/Kg/day | 9 | 35.0 ± 0.8 | 42.2 ± 0.9 |

Test Example 2

Measurment for nerve conduction velocity in GK rats with spontaneous diabetes

In similar way to Test Example 1, variations in nerve conduction velocity were measured for (17S)-17,20-dimethylisocarbacyclin methyl ester [=(17S)-17,20-dimethyl-9(0)-methano-$\Delta^{6(9\alpha)}$-prostaglandin $I_1$ methyl ester as test substance. The results are shown in Table 2.

Improvements on nerve conduction velocity were observed on both the groups administered at 30µg/Kg/day and 300µg/Kg/day of the test substance after 4 weeks, in comparison with the control group of GK rats.

Table 2

Variations in nerve conduction velocity before administration of test substance and 4 weeks later

| Animals | | Nerve conduction velocity (m/sec) | |
|---|---|---|---|
| | Numbers | Before administration | 4 weeks later |
| Control (Wistar rats) | 10 | 32.2 ±0.8 | 33.9 ±0.5 |
| Control (GK rats) | 7 | 29.5 ±1.0 | 31.8 ±1.0 |
| GK rats 30µg/Kg/day | 9 | 30.9 ±1.0 | 38.1 ±1.0 |
| GK rats 300µg/Kg/day | 7 | 32.4 ±0.7 | 37.5 ±1.4 |

Example 1

Tablets were prepared each with the following composition.

| | |
|---|---|
| Active ingredient | 100 µg |
| Lactose | 280 mg |
| Potato starch | 80 mg |
| Polyvinyl pyrrolidone | 11 mg |
| Magnesium stearate | 5 mg |

The active ingredient, lactose and potato starch were mixed together and uniformly wetted with 20% ethanol solution of polyvinyl pyrrolidone. The mixture was passed through a 20 mesh screen, dried at 45°C and passed through a 15 mesh screen to obtain granules, which were then kneaded with magnesium stearate and compressed into tablets.

The active ingredient used was (17S)-17,20-dimethylisocarbacyclin as a typical example.

Example 2

Tablets were prepared each with the following composition.

| | |
|---|---|
| Active ingredient | 500 µg |
| Lactose | 280 mg |
| Potato starch | 80 mg |
| Polyvinyl pyrrolidone | 11 mg |
| Magnesium stearate | 5 mg |

The active ingredient, lactose and potato starch were mixed together and uniformly wetted with 20% ethanol solution of polyvinyl pyrrolidone. The mixture was passed through a 20 mesh screen, dried at 45°C and passed through a 15 mesh screen to obtain granules, which were then kneaded with magnesium stearate and compressed into tablets.

The active ingredient used was (17S)-17,20-dimethylisocarbacyclin as a typical example.

Example 3

Hard gelatin capsules were prepared each containing the following components.

| | |
|---|---|
| Active ingredient | 100 µg |
| Microcrystalline cellulose | 195 mg |
| Amorphous silicic acid | 5 mg |

The active ingredient in the form of fine particles, microcrystalline cellulose and amorphous sililic acid were thoroughly mixed together and packed into hard gelatin capsules.

The active ingredient used was the same compound as used in Example 1 as a typical example.

Example 4

The same active ingredient as used in Example 1 was dissolved in fractionated coconut oil to obtain an oily solution having 2 mg/g concentration.

By using a serious automatic soft capsule machine, the oily solution was applied with a coating agent (100 parts (by weight) of gelatin, 20 parts of glycerin, 0.2 parts of ethyl p-oxybenzoate, 0.2 parts of propyl p-oxybenzoate and 80 parts of purified water) to form and dry soft capsules, each containing 200 µg of the active ingredient.

Example 5

Tablets were prepared each with the following composition.

| | |
|---|---|
| Active ingredient | 100 µg |
| Lactose | 280 mg |
| Potato starch | 80 mg |
| Polyvinyl Pyrrolidone | 11 mg |
| Magnesium stearate | 5 mg |

The active ingredient, lactose and potato starch were mixed together and uniformly wetted with 20% ethanol solution of polyvinyl pyrrolidone. The mixture was passed through a 20 mesh screen, dried at 45°C and passed through a 15 mesh screen to obtain granules, which were then kneaded with magnesium stearate and compressed into tablets.

The active ingredient used was (17S)-17,20-dimethylisocarbacyclin methyl ester as a typical example.

Example 6

Tablets were prepared each with the following composition.

| | |
|---|---|
| Active ingredient | 500 µg |
| Lactose | 280 mg |
| Potato starch | 80 mg |
| Polyvinyl pyrrolidone | 11 mg |
| Magnesium stearate | 5 mg |

The active ingredient, lactose and potato starch were mixed together and uniformly wetted with 20% ethanol solution of polyvinyl pyrrolidone. The mixture was passed through a 20 mesh screen, dried at 45 °C and passed through a 15 mesh screen to obtain granules, which were then kneaded with magnesium stearate and compressed into tablets.

The active ingredient used was (17S)-17,20-dimethylisocarbacyclin methyl ester as a typical example.

Example 7

Hard gelatin capsules were prepared each containing the following components.

| | |
|---|---|
| Active ingredient | 100 µg |
| Microcrystalline cellulose | 195 mg |
| Amorphous silicic acid | 5 mg |

The active ingredient in the form of fine particles, microcrystalline cellulose and amorphous sililic acid were thoroughly mixed together and packed into hard gelatin capsules.

The active ingredient used was the same compound as used in Example 5 as a typical example.

Example 8

The same active ingredient as used in Example 5 was dissolved in fractionated coconut oil to obtain an oily solution having 2 mg/g concentration.

By using a serious automatic soft capsule machine, the oily solution was applied with a coating agent (100 parts (by weight) of gelatin, 20 parts of glycerin, 0.2 parts of ethyl p-oxybenzoate, 0.2 parts of propyl p-oxybenzoate and 80 parts of purified water) to form and dry soft capsules, each containing 200 µg of the active ingredient.

## Claims

**1.** A use of an isocarbacyclin derivative of the formula (I):

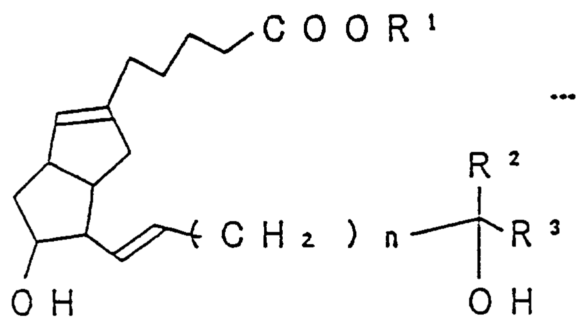

... [ I ]

wherein $R^1$ is hydrogen atom, an alkyl group or one equivalent of cation; $R^2$ is hydrogen atom or methyl group; $R^3$ is a straight-chain or branched-chain alkyl, alkenyl or alkynyl group, a cycloalkyl, phenyl or phenoxy group which may be substituted, or a straight-chain or branched-chain alkyl group which is

substituted by the above mentioned cycloalkyl, phenyl or phenoxy group which may be substituted; n is 0 or 1, provided that when $R^3$ is n-pentyl, $R^2$ is methyl or n is 1, for the manufacture of a medicament for therapeutic treatment of diabetic neuropathy.

**2.** The use according to claim 1 wherein $R^1$ is hydrogen atom or methyl group.

**3.** The use according to claim 1 wherein $R^2$ is hydrogen atom.

**4.** The use according to claim 1 wherein $R^3$ is a straight-chain or branched-chain alkyl group having 3 to 10 carbon atoms.

**5.** The use according to claim 1 wherein $R^3$ is 2-methyl-hexyl group.

**6.** The use according to claim 1 wherein n is 0.

**7.** The use according to claim 1 wherein the isocarbacyclin derivative is (17S)-17,20-dimethyl-9(0)-methano-$\Delta^{6(9\alpha)}$-prostaglandin $I_1$.

**8.** The use according to claim 1 wherein the isocarbacyclin derivative is (17S)-17,20-dimethyl-9(0)-methano-$\Delta^{6(9\alpha)}$-prostaglandin $I_1$ methyl ester.

**Patentansprüche**

**1.** Verwendung eines Isocarbacyclin-Derivates der Formel (I):

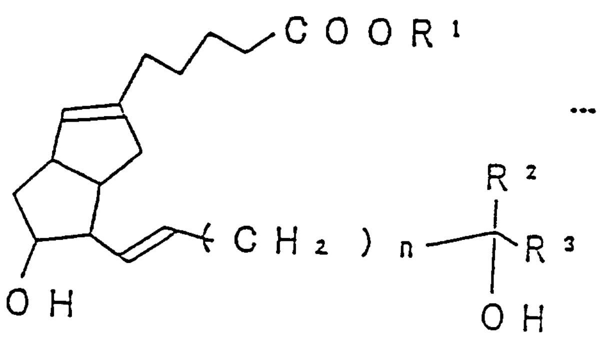

··· [ I ]

worin $R^1$ ein Wasserstoffatom, eine Alkylgruppe oder ein Äquivalent eines Kations darstellt; $R^2$ ein Wasserstoffatom oder eine Methylgruppe darstellt; $R^3$ eine geradkettige oder verzweigtkettige Alkyl-, Alkenyl- oder Alkinylgruppe, eine Cycloalkyl-, Phenyl- oder Phenoxygruppe, die substituiert sein kann, oder eine geradkettige oder verzweigtkettige Alkylgruppe darstellt, die durch die oben ervähnte Cycloalkyl-, Phenyl- oder Phenoxygruppe, die substituiert sein kann, substituiert ist; n 0 oder 1 darstellt, mit der Maßgabe, daß wenn $R^3$ n-Pentyl ist, $R^2$ Methyl ist oder n 1 ist, zur Herstellung eines Medikamentes zur therapeutischen Behandlung der diabetischen Neuropathie.

**2.** Verwendung nach Anspruch 1, worin $R^1$ ein Wasserstoffatom oder eine Methylgruppe darstellt.

**3.** Verwendung nach Anspruch 1, worin $R^2$ ein Wasserstoffatom darstellt.

**4.** Verwendung nach Anspruch 1, worin $R^3$ eine geradkettige oder verzweigtkettige Alkylgruppe mit 3 bis 10 Kohlenstoffatomen darstellt.

**5.** Verwendung nach Anspruch 1, worin $R^3$ eine 2-Methylhexyl-Gruppe darstellt.

**6.** Verwendung nach Anspruch 1, worin n 0 darstellt.

**7.** Verwendung nach Anspruch 1, worin das Isocarbacyclin-Derivat (17S)-17,20-Dimethyl-9(0)-methano-$\Delta^{6(9\alpha)}$-prostaglandin $I_1$ ist.

8. Verwendung nach Anspruch 1, worin das Isocarbacyclin-Derivat (17S)-17,20-Dimethyl-9(0)-methano-$\Delta^{6(9\alpha)}$-prostaglandin $I_1$-Methylester ist.

**Revendications**

1. Utilisation d'un dérivé d'isocarbacycline répondant à la formule (I) :

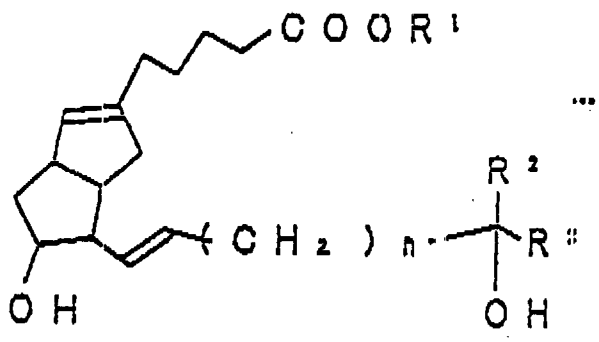

... [ I ]

dans laquelle $R^1$ est un atome l'hydrogéne, un groupe alkyle ou un équivalent d'un cation; $R^2$ est un atome d'hydrogène ou un groupe méthyle; $R^3$ est un groupe alkyle, alcényle ou alcynyle à chaîne linéaire ou à chaîne ramifiée, un groupe cycloalkyle, phényle ou phénoxy qui peut être substitué, ou un groupe alkyle à chaîne linéaire ou à chaine ramifiée qui est substitué par le groupe cycloalkyle, phényle ou phénoxy qui peut être substitué, mentionné ci-dessus; n est 0 ou 1, à condition que, lorsque $R^3$ est un groupe n-pentyle, $R^2$ soit un groupe méthyle ou n soit 1, pour la fabrication d'un médicament pour le traitement thérapeutique de la neuropathie diabétique.

2. Utilisation selon la revendication 1, dans laquelle $R^1$ est un atome d'hydrogène ou un groupe méthyle.

3. Utilisation selon la revendication 1, dans laquelle $R^2$ est un atome d'hydrogène.

4. Utilisation selon la revendication 1, dans laquelle $R^3$ est un groupe alkyle à chaîne linéaire ou à chaîne ramifiée ayant 3 à 10 atomes de carbone.

5. Utilisation selon la revendication 1, dans laquelle $R^3$ est un groupe 2-méthyl-hexyle.

6. Utilisation selon la revendication 1, dans laquelle n est 0.

7. Utilisation salon la revendication 1, dans laquelle le dérivé d'isocarbacycline est la (17S)-17,20-diméthyl-9(0)-méthano-$\Delta^{6(9\alpha)}$-prostaglandine $I_1$.

8. Utilisation selon la revendication 1, dans laquelle le dérivé d'isocarbacycline est l'ester méthylique de la (17S)-17,20-diméthyl-9(0)-méthano-$\Delta^{6(9\alpha)}$-prostaglandine $I_1$.